# EUROPÄISCHE PATENTSCHRIFT

(11) **EP 1 090 984 B1**
(45) Veröffentlichungstag und Bekanntmachung des Hinweises auf die Patenterteilung: **18.12.2002**
(21) Anmeldenummer: 00119552.8
(22) Anmeldetag: 07.09.2000
(51) Int. Cl.: C12M 3/06

(54) **Verfahren zur Erzeugung, zum Transport und zur Eliminierung von biochemischen Stoffen mittels aktiver biologischer Zellen**
Process for the production, the transport and the elimination of biochemical products with biological active cells
Procédé pour la production, le transport et l'élimination de composés biochimiques à l'aide de cellules biologiques actives

(30) Priorität: 10.09.1999 DE 19943205
(43) Veröffentlichungstag der Anmeldung: 11.04.2001
(73) Patentinhaber: GKSS-FORSCHUNGSZENTRUM GEESTHACHT GMBH, 21502 Geesthacht (DE); GROSS, Ulrich, Prof. Dr., 14195 Berlin (DE)
(72) Erfinder: Albrecht, Wolfgang, Dr., 14513 Teltow (DE); Groth, Thomas, Dr., 10439 Berlin (DE); Seifert, Barbara, Dr., 10249 Berlin (DE); Paul, Dieter, Prof. Dr., 14532 Kleinmachnow (DE); Prof. Dr. Ulrich Gross, 14195 Berlin (DE); Dr. Frédérique Fey-Lamprecht, 85521 Ottobrunn (DE)
(74) Vertreter: Niedmers, Ole, Dipl.-Phys.

(56) Entgegenhaltungen:
- WO-A-89/01967
- WO-A-96/09876
- US-A- 5 686 289

## Beschreibung

Die Erfindung betrifft ein Verfahren zur Erzeugung, zum Transport und zur Eliminierung von biochemischen Stoffen mittels aktiver biologischer Zellen.

Es ist bekannt, daß mit wenigen Ausnahmen alle inneren und äußeren Oberflächen von höheren Lebewesen, beispielsweise des menschlichen Körpers, mit einer polar organisieren Schicht von Zellen ausgekleidet sind, die ein Epithel bilden. Die einzelnen Epithelien haben jeweils besondere Strukturen, die für eine koordinierte Funktion und Integrität notwendig sind. Die Epithelzellen weisen u.a. besondere Trenneigenschaften auf, die im lebenden Körper von Menschen und Tieren Grundlage der Funktion von Organen wie Niere, Leber, Darm, Bauchspeicheldrüse und anderen sind. Sie bestimmen die Resorptions - (Filter-) und Sekretionseigenschaften der Organe. Bei eingeschränkter Funktion oder Ausfall dieser Organe ist versucht worden, bestimmte Funktionen dieser Organe mit Hilfe von technischen Vorrichtungen zu simulieren, um bei Organschädigung oder - versagen die Überlebenschancen des Menschen bzw. des Tieres zu erhöhen.

Ein Beispiel dafür ist die sogenannte künstliche Niere, die schon seit Jahren zur Herausfilterung von Schadstoffen aus dem Blut eingesetzt wird. Bisher wurde dabei jedoch nur der Filtrationsprozeß der glomerulären Membranen mit Hilfe von künstlichen Membranen mit bestimmten Trenneigenschaften simuliert. Dieser Prozeß führt jedoch zu einer unspezifisch, partiellen Abtrennung aller Komponenten des Blutplasmas bis zu einem Molekulargewicht von 60.000 Dalton. Die Sekretion bzw. Rückresorption verschiedener Stoffe, die von den Nierenzellen Epithelzellen realisiert werden, konnte damit prinzipiell nicht erreicht werden. Damit verbunden sind hohe Verluste an Wasser, Ionen, Glukose und von Proteinen kleinen Molekulargewichts.

Man hat versucht, dieses Problem durch die Entwicklung von biohybriden Organen (BHO) oder bioartifiziellen Organen (BAO) zu lösen, in denen die vorab beschriebene Filtration durch Dialysemembranen mit einer Leistung immobilisierter Epithelzellen verbunden wird.

Es wurden daher vielfältige Versuche unternommen, sogenannte Bioreaktoren, zu entwickeln. Für die Immobilisierung benötigen die Epithelzellen ein die Basallamina simulierendes Substrat, auf dem sie sich anheften und ihre Polarität entwickeln können. Dabei müssen sowohl die Kontakte zwischen Unterlage und Zellen als auch zwischen den Zellen gut ausgebildet sein. Es hat sich aber gezeigt, daß dabei verwendete Supporte/Membranen oftmals nur eine ungenügende Unterstützung der Haftung und Funktion der Epithelzellen ermöglichen.

Es sind unterschiedliche Bioreaktoren bekannt, in denen lebende Zellen (aber auch Enzyme) zur Erzeugung und zum Transport von Stoffen eingesetzt werden. Im einfachsten Falle werden dabei Zellsuspensionen in einem thermostatisierten und begasten Gefäß mit entsprechenden Medien unter Bewegung (Rührvorrichtung) kultiviert. Nach einer gewissen Zeit werden die Zellen und entstandene Produkte entfernt und die Produkte durch aufwendige physikalisch-chemische und andere Methoden isoliert und konzentriert. In Reaktoren mit immobilisierten Zellen sind die lebenden Zellen an Trägerpartikel gebunden (attachment, sorption), in eine poröse Matrix (Gel) eingebettet, und/oder hinter einer Barriere (Mikrokapsel, Membran) eingeschlossen. Dadurch ist ein quasi kontinuierlicher Prozeß realisierbar, bei dem die Zellen im Reaktor verbleiben, während die Eingangs- und Ausgangsstoffe zu- bzw. abgegeben werden und, falls erwünscht, konstante Stoffkonzentrationen eingestellt werden können.

Auch Bioreaktoren, die Dreikompartimentsysteme mit Nährlösungsraum, Zellkulturraum und Produktraum benutzen, sind bereits in der Literatur beschrieben (z.B. Applied Microbiology; Differential Dialysis Culture for Separation and Concentration of Macromolecular Product, 15 (1967), 1192-1197 oder J. Membrane Sci.: Membrane bioreactor for lactic acid production 114 (1996), 59-71), um das Produkt der Zellreaktion aus dem Zeitraum in den Produktraum zu überführen. Die Räume sind hierbei mit Membranen unterschiedlicher Permeabilität getrennt.

Einerseits werden in diesen Reaktoren keine sich polar organisierenden Zellen im Zeitraum verwendet, was andererseits bedingt, daß kein gerichteter Transport stattfinden kann. Hier bestimmen insbesondere das Trennverhalten der im Reaktor eingesetzten Membranen und die experimentellen Bedindungen des Reaktorbetriebes, welcher Stoff sich entsprechend seines Konzentrationsgradienten in welchem Raum des Bioreaktors befindet. Man spricht von einem passiven Transport. Es ist lediglich sichergestellt, daß entsprechend der gewählten Arbeitsbedingungen die Zellen im Zellraum verbleiben. Ein gegen den Gradienten erfolgender aktiver Transport ist unter Verwendung dieser Bioreaktoren nicht möglich.

WO-A-89/01967 offenbart ein Verfahren zur Simulierung der Nierenfunktion, bei dem das Blut durch zwei getrennte und mit verschiedenen Nierenzellen gefüllten Hohlfadenbioreaktoren filtriert wird. US-A-5,686,289 offenbart ein Hohlfadenbioreaktor, bei dem verschiedene Nierenzellen in verschiedenen Hohlfäden eingebracht werden.

Nahezu alle bisher bekannten Bioreaktortypen, die sich mit dem eingangs genannten Verfahren bzw. ähnlichen Verfahren befassen, wurden aber nicht für einen gerichteten, aktiven Stofftransport durch Zellen konzipiert. So wachsen Zellen nicht in epithelähnlichen Verbänden, was jedoch für den Aufbau von Stoffgradienten unerläßlich ist bzw. deren Konstruktion ist nicht geeignet, Gradienten von Stoffen aufzubauen und diese durch Zellaktivität verändern zu lassen.

Es ist somit Aufgabe der vorliegenden Erfindung, ein Verfahren zu schaffen, mit dem die Kultivierung und Nutzung von biologischen Zellen bzw. biologischen Systemen für Entgiftungs-, Erzeugungs- und Transportprozesse von biologischen und anderen Substanzen möglich ist, wobei Zellen derart kultiviert werden sollen, daß eine polare Organisation von epithelähnlichen Zellschichten ermöglicht wird und die Zellen für die Entgiftungs-, Herstellungs- und Transportprozesse genutzt werden können, wobei das Verfahren kontinuierlich für biotechnologische und biomedizinische Anwendungen genutzt werden kann.

Gelöst wird die Aufgabe gemäß der Erfindung durch folgende Schritte:
a. Einbringen von sich polar organisierenden Zellen in einen ersten Raum, der durch wenigstens zwei Membranen unterschiedlicher Permeabilität begrenzt ist,
b. kontinuierliches Einbringen einer Nährlösung in einen zweiten und/oder dritten Raum zum am Leben erhalten der Zellen und zur Vermehrung der Zellen, (Proliferationsphase der Zellen und Ausbildung der polar organisierten Zellschicht bzw. Zellschichten),
c. Führung eines mit Fremdstoff angereicherten Mediums durch einen zweiten oder dritten Raum in Kontakt mit einer der beiden Membranen, die den ersten Raum vom zweiten Raum oder den ersten Raum vom dritten trennt, und eines zweiten Aufnahme-/Entgiftungsmediums durch den dritten oder zweiten Raum in Kontakt mit einer der beiden Membranen, die den ersten Raum vom dritten Raum oder den ersten vom zweiten Raum trennt, wobei durch aktive Zellfunktion der Zellen der Fremdstoff durch die Membran in den ersten Raum überführt und durch Zellstoffwechsel derart behandelt wird, daß
d. nachfolgend durch den Zellstoffwechsel erzeugte niedermolekulare Stoffe durch die andere der beiden Membranen, die den ersten Raum vom zweiten oder dritten Raum trennt, durch aktive Zellfunktion der Zellen überführt wird, wohingegen
e. durch den Zellstoffwechsel erzeugte oder entgiftete hochmolekulare Stoffe über die entgegengesetzte Membran, die den ersten Raum vom zweiten oder dritten Raum trennt, durch aktive Zellfunktion in den zweiten oder dritten Raum in das mit Fremdstoff belastete Medium zurücküberführt werden (Arbeitsphase).

Der Vorteil der erfindungsgemäßen Lösung besteht im wesentlichen darin, daß nunmehr die Ausbildung beispielsweise funktionstüchtiger Epithelzellschichten in dem von zwei Membranen begrenzten ersten Raum möglich ist, durch die eine gerichteter, aktiver Stofftransport wenigstens bei einer der Membranen in beiden Richtungen durch die Membran hindurch möglich ist, und zwar auch gegen bestehende Konzentrationsgefälle. Die in dem Raum zwischen den beiden Membranen nach dem Einbringen in diesen Raum sich befindenden immobilisierten Zellen (Epithelzellen) sind so angeordnet, daß ihre polare Funktion bezüglich apikaler und basaler Seite bestehen bleibt, d.h., sie bilden ein intaktes abschließendes Epithel, das diesen Gradienten erzeugt bzw. aufrechterhält.

Das erfindungsgemäße Verfahren eignet sich somit für den therapeutischen Einsatz als biohybrides Organ wie bei einer biohydriden Niere, Leber oder Pancreas. Auch ist das erfindungsgemäße Verfahren zur Herstellung/Erzeugung und Konzentration von Stoffen von Drüsenzellen, sowie zur in-vitro-Erzeugung von Hormonen, Speichel, Milch und anderen Sekreten nutzbar. Schließlich ist damit die Nutzung zur Konzentrierung von Stoffen aus verdünnten Lösungen möglich.

Gemäß einer vorteilhaften Ausgestaltung des Verfahrens wird die Nährlösung mit Sauerstoff und/oder Kohlendioxyd beaufschlagt, um optimale Lebensbedingungen für die Zellen im ersten Raum zwischen den Membranen zu gewährleisten.

Es hat sich gezeigt, daß das Verfahren die besten Ergebnisse zeigt, wenn die sich polar organisierenden Zellen im ersten Raum eine möglichst dünne Schicht, angestrebt wird vorzugsweise eine lediglich einmolekular dicke Schicht, bilden, wobei die Zellen vorzugsweise den ersten Raum wenigstens teilweise ausfüllen.

Vorteilhafterweise ist die erste Membran, die mit dem mit den Fremdstoffen angereichertem Medium kontaktiert wird, mikroporös, wobei vorzugsweise die erste Membran, die mit dem mit den Fremdstoffen angereichertem Medium kontaktiert wird, mit ihrer trennaktiven Schicht dem Medium zugewandt ist.

In Anknüpfung an das oben Gesagte im Zusammenhang mit der angestrebten möglichst dünnen Schicht der Zellen im ersten Raum zwischen den beiden Membranen ist es auch vorteilhaft, die Querschnittsfläche des ersten Raumes, der zwischen den beiden Membranen eingeschlossen ist, < 10 Zellendurchmesser auszubilden, um die Diffusion und/oder Konvektion der Fremdstoffe im Medium zu den Zellen im ersten Raum und die in den Zellen erzeugten oder entgifteten Stoffe wiederum in den zweiten Raum oder dritten Raum in das Medium zurück bzw. Stoffwechselprodukte der Zellen in den dritten oder zweiten Raum hinein unter günstigsten Bedingungen zu ermöglichen.

Zur Ausführung des erfindungsgemäßen Verfahrens ist grundsätzlich die konstruktive Art der verwendeten Membran bzw. der verwendeten Membranen ohne Einfluß auf die grundsätzliche Funktionsfähigkeit des Verfahrens. So kann es beispielsweise vorteilhaft sein, beide Membranen, die den ersten Raum einschließen, in dem sich die Zellen befinden, als Flachmembranen auszubilden oder bei einer anderen vorteilhaften Ausgestaltung eine Membran als Hohlfadenmembran und die andere Membran als Flachmembran auszubilden. Dieses wird letztlich von der Ausbildung und/oder Verwendung einer Vorrichtung abhängig sein, mit der das erfindungsgemäße Verfahren ausgeführt werden soll und somit auch an den unmittelbaren Verwendungszweck angepaßt sein soll.

Es ist vorteilhafterweise auch möglich, beide Membranen als Hohlfadenmembranen auszubilden, wobei der erste Raum zwischen der äußeren Membranfläche einer ersten Hohlfadenmembran und der äußeren Membranfläche einer zweiten Hohlfadenmembran gebildet wird.

Besonders vorteilhaft ist es, beide Membranen als Hohlfadenmembranen auszubilden, wobei der erste Raum zwischen der äußeren Membranfläche einer ersten Hohlfadenmembran und der inneren Membranfläche einer zweiten Hohlfadenmembran gebildet wird und diese dann noch so anzuordnen, daß die beiden Hohlfadenmembranen im wesentlichen koaxial ineinander angeordnet sind. Die letzte Ausgestaltung gestattet einen sehr kompakten Aufbau einer Vorrichtung zur Ausführung des Verfahrens, wobei bei einer derartigen Ausgestaltung auch die vorangehend aufgeführten optimalen Verfahrensparameter auf einfache Weise realisierbar sind. Man spricht in diesem Zusammenhang auch von einem Hohlfaden-in-Hohlfaden Aufbau (Hohlfaden-in-Hohlfaden-Bioreaktor).

Die Membranen selbst sind vorteilhafterweise Polymermembranen, bei denen, je nach Anwendungszweck, unterschiedliche Membranstrukturen und Membranparameter, wie Membranpermeabilität und Membranfluß usw. auf vorbestimmbare Weise eingestellt werden können. Dieses wird von der Verwendung bzw. Anwendung des Verfahrens in Bezug auf das mit Fremdstoffen angereicherte Medium sowie die polar orientierten Zellen im ersten Raum zur Behandlung der Fremdstoffe im Medium bzw. des Mediums selbst abhängen.

Die Polymermembranen sind vorzugsweise Membranen aus Polyacrilnitril (PAN) oder Polysolfon (PSU).

Wenigstens die Verfahrensschritte, die im eigentlichen Bioreaktor, d.h. dem ersten Raum, in dem die polar sich organisierenden Zellschichten angeordnet sind, im zweiten oder dritten Raum, in dem das zu behandelnde Medium strömt, und in einem zweiten oder dritten Raum, in dem die niedermolekularen Metabolite der Bioreaktion beispielsweise aus toxischem Blut oder Bestandteilen desselben durch die Membran hindurchgeführt, in eine Spülflüssigkeit überführt und abgeführt wird, werden bei vorwählbarer, stabil haltbarer Temperatur durchgeführt, beispielsweise bei einer Temperatur von 37° C.

Die Erfindung wird nun unter Bezugnahme auf die nachfolgenden schematischen Zeichnungen sowie Darstellungen bezüglich der Aktivität unterschiedlicher Zellen nach unterschiedlichen Tagen der Kultivierung auf der dichten Seite der Membranen und der porösen Seite der Membranen bei unterschiedlichen Membranpolymeren beschrieben. Darin zeigen:
- Fig. 1: schematisch den Aufbau einer Vorrichtung zur Ausführung des Verfahrens,
- Fig. 2: das Beispiel einer Schaltung, in der die Vorrichtung gemäß Fig. 1 (Bioreaktor) eingebunden ist,
- Fig. 3: die mitochondriale Aktivität (MTT-Test) von MDCK-Zellen nach verschiedenen Tagen der Kultivierung auf der dichten Seite der Membranen,
- Fig. 4: die mitochondriale Aktivität (MTT-Test) von MDCK-Zellen nach verschiedenen Tagen der Kultivierung auf der porösen Seite der Membranen,
- Fig. 5: die Laktat-Dehydrogenase-Aktivität (LDH-Test) von MDCK-Zellen nach verschiedenen Tagen Kultivierung auf der dichten Seite der Membranen,
- Fig. 6: Laktatdehydrogenase-Aktivität (LDH-Test von MDCK-Zellen nach verschiedenen Tagen der Kultivierung auf der porösen Seite der Membranen,
- Fig. 7: eine TEM-Aufnahme von MDCK-Zellen auf der dichten Seite der Membranen (3000 x bzw. 1000 x) nach 7 Tagen Kultur für eine PAN-Membran,
- Fig. 8: Aufnahme gemäß Fig. 7, jedoch bei einer PSU-Membran,
- Fig. 9: eine Darstellung des transephitelialen Widerstandes von MDCK-Zellen nach 7 Tagen Kultivierung auf der dichten Seite der Membranen,
- Fig. 10: die mitochondriale Aktivität (MTT-Test) von C3A-Zellen nach verschiedenen Tagen der Kultivierung auf der dichten Seite der Membranen,
- Fig. 11: die mitochondriale Aktivität (MTT-Test) von C3A-Zellen nach verschiedenen Tagen Kultivierung auf der porösen Seite der Membranen,
- Fig. 12: Laktatdehydrogenase-Aktivität (LDH-Test) von C3A-Zellen nach verschiedenen Tagen Kultivierung auf der dichten Seite der Membranen,
- Fig. 13: Laktatdehydrogenase- Aktivität (LDH-Test) von C3A-Zellen nach verschiedenen Tagen Kultivierung auf der porösen Seite der Membranen,
- Fig. 14: eine TEM-Aufnahme von C3A-Zellen auf der dichten Seite der Membranen (3000 x) nach 7 Tagen Kultur für eine PAN-Membran,
- Fig. 15: Aufnahme gemäß Fig. 14, jedoch bei einer PSU-Membran,
- Fig. 16: eine Darstellung der Albuminproduktion von C3A-Zellen zwischen dem 8. und 9. Kultivierungstag,
- Fig. 17: eine Darstellung des Verlaufes der Albuminproduktion in einem Bioreaktor mit PAN-Fäden durch C3A-Zellen und
- Fig. 18: den Verlauf der Albuminproduktion im Bioreaktor mit PSU-Fäden durch C3A-Zellen.

Bevor nachfolgend im einzelnen auf das Verfahren und eine Vorrichtung 10 (Bioreaktor) eingegangen wird, mit der das Verfahren ausgeführt werden kann, wird auf die Zellen bzw. den Zellstoffwechsel, beispielsweise von Ephitelzellen, eingegangen, die bzw. der bei dem Verfahren genutzt wird. Die Plasmamembran einer polaren Zelle ist in zwei Regionen organisiert, nämlich in eine apikale und eine basale, die jeweils bestimmte Eigenschaften besitzen. Die Zwischenräume zwischen polaren Zellen werden an der apikalen Seite mit sogenannten "Tight-Junctions" (zonulae occludentes) versiegelt, von deren Unversehrtheit die funktionelle Integrität eines Gewebes ganz wesentlich abhängt. In den darunter liegenden Abschnitten der Zellzwischenräume vermitteln Desmosomen und "Gap-Junctions" den Zellverbund. Die "Tight-Junctions" in den Zwischenräumen trennen das apikale und basale Kompartment ab und sind für Proteine, Lipide und Ionen weitgehend undurchlässig. Ein Transport von Stoffen kann deshalb nur durch die Zellen hindurch erfolgen und ist deshalb von der zelltypischen Aktivität abhängig, was durch eine Reihe von Prozessen, wie der Pinozytose und dem aktiven Ionen- bzw. Kationentransport über sogenannte Protonen-Pumpen erfolgen kann.

Die apikale Zellmembranregion ist nach der Außenseite, dem Lumen, gerichtet und oft mit zahlreichen fingerförmigen Ausläufen (mikrovilli) ausgestattet, die u.a. den selektiven Stoffaustausch mit dem Lumen realisieren und gleichzeitig die Membranoberfläche vergrößern.

Die basallaterale Zellmembranregion besteht aus der basalen Membran (im Kontakt mit der extrazellulären Matrix auf der Basallamina, die kapillare Blutgefäße enthält) und der lateralen Membran zwischen den Zellen. Dieser Membranabschnitt ist für die Stabilität und Haftung der Zelle verantwortlich, stellt aber keine Barriere für den transephitelialen Transport dar. Der gerichtete transephiteliale Transport von der apikalen zur basallateralen Seite der Ephitelzelle wird Resorption genannt, in entgegengesetzte Richtung Sekretion.

Die hier kurz dargestellten Trenneigenschaften von Epithelzellen sind im lebenden Körper Grundlage der Funktion von Organen wie Leber, Niere, Darm, Bauchspeicheldrüse e.a. und bestimmen, wie eingangs schon angedeutet, die Resorptions- (Filter-) und Sekretionseigenschaften der Organe.

Mit Hilfe des hier vorgeschlagenen Verfahrens können beispielsweise bestimmte Funktionen dieser Organe mit Hilfe von Vorrichtungen, wie sie anhand einer beispielhaften Vorrichtung 10 beschrieben wird, simuliert werden, um bei Organschädigung oder -versagen die Überlebenschancen eines Patienten zu erhöhen.

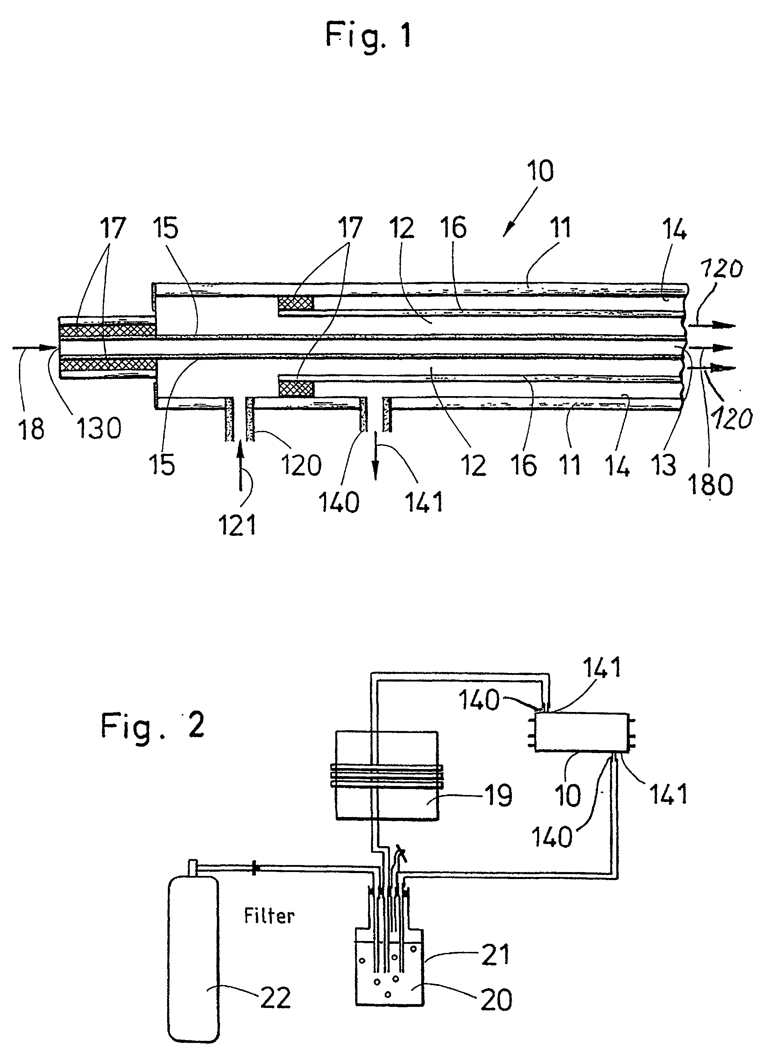

Es wird Bezug genommen auf die Darstellung gemäß Fig. 1, in der eine Vorrichtung 10 bezüglich ihres schematischen Aufbaus dargestellt ist, mit der das hier beschriebene Verfahren ausgeführt werden kann. Die Strömungsführung im Betrieb wird für ein Beispiel in der Arbeitsphase beschrieben.

Die Vorrichtung 10 zur Ausführung des Verfahrens umfaßt drei Räume 12, 13, 14, die hier - lediglich beispielhaft - im wesentlichen koaxial zueinander angeordnet sind, wobei die Vorrichtung 10 von einem Gehäuse 11 umschlossen ist. Im Gehäuse 11 sind zwei in Form von Hohlfadenmembranen ausgebildete Membranen, erste Membran 15 und zweite Membran 16, ausgebildet. Die erste und die zweite Membran 15 und 16 sind koaxial zueinander ausgebildet. Das Gehäuse 11 weist im wesentlichen zentral bzw. axial die erste Membran 15 auf, in deren Innenraum ein zweiter Raum 13 gebildet wird, der von einem mit Fremdstoffen angereicherten Medium 18 durchflossen wird. Das Medium tritt, von Fremdstoff aufgrund der verfahrensmäßigen Behandlung partiell oder vollständig befreit, aus der Vorrichtung 10 als Medium 180 aus. Im wesentlichen koaxial zur ersten Membran 15 ist eine zweite Membran 16 angeordnet, wobei zwischen erster und zweiter Membran ein erster Raum 12 gebildet wird, in dem die in die Vorrichtung 10 eingebrachten, sich polar organisierenden Zellen angeordnet sind.

Zwischen der zweiten Membran und dem Gehäuse 11 wird ein dritter Raum 14 gebildet, der Aufnahmeraum für abgeführte Stoffe ist, die durch den Zellstoffwechsel erzeugt und in den dritten Raum 14 überführt und über eine Abführöffnung 140 als abgeführter Stoff 141 aus der Vorrichtung 10 entfernt werden, ggf. unterstützt durch ein Spülmittel, das durch den dritten Raum 14, ggf. kontinuierlich, geführt wird.

Die sich polar organisierenden Zellen werden als Suspension in einem Medium (121) über die Öffnung 120, die als Zufuhröffnung für den ersten Raum dient, in den Bioreaktor eingeführt. Die Suspension durchströmt den Raum 12, in dem die Zellen abgelagert (ausgesät) werden, und ein zellenarmes Medium 121 tritt über eine spiegelbildlich angeordnete Abführöffnung 120 aus der Vorrichtung 10 aus.

Alle Räume 12, 13, 14 sind gegeneinander und nach außen hermetisch durch Dichtungen 17 abgedichtet.

In einer tatsächlich realisierten Vorrichtung 10 befinden sich eine Mehrzahl von derart mit den Membranen 15, 16 bzw. den Räumen 12, 13 und 14 ausgebildeten Modulen mit jeweils einer Mehrzahl von derart angeordneten Membranpaaren, die dem zu erwartenden Aufkommen an zu behandelndem Medium sowie der Zellen und anderer Verfahrensparameter qualitativ und quantitativ angepaßt sind. Das vorbezeichnete grundsätzliche Schema der Vorrichtung 10 wird dabei aber nicht verlassen.

Die in Fig. 2 dargestellte Schaltung, in der die Vorrichtung 10 (Bioreaktor) eingeschaltet ist, umfaßt eine Pumpe 19, mit der das Nähr-/Aufnahme-/Entgiftungsmedium aus einem Behälter 21, in dem sich das genannte bzw. die genannten Medium bzw. Medien 141 befindet bzw. befinden, in die Vorrichtung 10 über die Zufuhröffnung 140 gepumpt und über das einer entsprechenden Abführöffnung 140 erneut in den Behälter 21 zurückgeführt und von dort entsprechend aufbereitet erneut in die Vorrichtung 10 gepumpt. Mit dem Behälter 21 bzw. der Nährlösung 121 ist zudem eine Gasversorgung 22 verbunden, die Sauerstoff und/oder Kohlendioxid in den Behälter 21 mit dem Nähr-/-Aufnahme-/Entgiftungsmedium 141 leitet. Mittels hier nicht gesondert dargestellter Temperierungs- und Regelungsmittel wird dafür gesorgt, daß alle wesentlichen Elemente, die an dem Verfahren mitwirken auf einer einstellbaren Temperatur, vorzugsweise 37° C, gehalten werden.

Das Verfahren läuft unter Benutzung der vorangehend beschriebenen Vorrichtung 10 sowie der Gesamtanlage gemäß Fig. 2 in folgenden Schritten ab:
a. Es werden zunächst polar sich organisierende Zellen in den ersten Raum 12 auf der ersten Membran 15 aufgebracht. Man spricht in diesem Zusammenhang von dem "Aussäen" der Zellen. Der erste Raum 12 ist durch die erste und zweite Membran 15, 16, die ein unterschiedliches Trennverhalten (Permeabilität,Fluß usw.) aufweisen, begrenzt.
b. Nachfolgend wird auf im Zusammenhang mit der in der Beschreibung von Fig. 2 beschriebenen Weise die Nährlösung 20 in den zweiten und/oder dritten Raum 13 zum am Leben erhalten und zur Vermehrung der Zellen eingebracht, und zwar kontinuierlich auf vorangehend beschriebene Weise. Man spricht in diesem Zusammenhang von der Proliferation der Zellen, wobei die polar organisierte Zellschicht im ersten Raum ausgebildet wird. Die Zellen im ersten Raum werden immobilisiert, um ein unkontrolliertes Wachstum weitgehend auszuschließen, das den mit dem Verfahren angestrebten Zellstoffwechsel negativ beeinflussen würde.
c. Nachfolgend wird ein mit Fremdstoff bzw. Fremdstoffen angereichertes Medium 18 über die Öffnung 130 in den zweiten Raum 13 geführt, der über die erste Membran 15 vom ersten Raum 12 hermetisch abgedichtet ist. Durch aktive Zellfunktion der Zellen wird der Fremdstoff durch die Membran 15 in den ersten Raum 12 überführt, d.h. der Stofftransport wird bzw. die Stoffumwandlung durch die Zellen selbst bewirkt.
d. Danach wird dann der durch den Zellstoffwechsel erzeugte niedermolekulare Stoff in den dritten Raum 14 überführt, der den Aufnahmeraum für abgeführte Stoffe bildet. Den Stofftransport für die abgeführten Stoffe übernehmen die Zellen im ersten Raum 12 wiederum selbst, und zwar aufgrund aktiver Zellfunktionen. Der Abtransport dieser niedermolekularen Stoffe aus dem Raum 14 des Bioreaktors 10 wird durch ein Aufnahme-/Entgiftungsmedium 141 unterstützt, das dem Raum 14 über die Zuführöffnung 141 zugeführt, den Raum 14 durchströmt und den Bioreaktor 10 über eine spiegelbildlich angeordnete Abführöffnung 141 verläßt.
e. Die durch den Zellstoffwechsel im ersten Raum 12 erzeugten hochmolekularen Stoffe werden wiederum über die erste Membran 15, wiederum durch aktive Zellfunktion initiiert, in den zweiten Raum 13 zurücküberführt, so daß das Medium als von Fremdstoffen 180 partiell oder vollständig befreit, aber um Stoffe wiederum angereichert, die durch die erste Membran 15 rückresorbiert werden, die Vorrichtung 10 als Medium 180 verläßt.

Mittels des hier beschriebenen Verfahrens kann unter Nutzung des vorangehend beschriebenen Bioreaktors gemäß Fig. 1 (Vorrichtung 10) der Aufbau eines Stoffgradienten zwischen den durch die Membranen 15, 16 jeweils ausgebildeten Räumen 12, 13, 14 ermöglicht werden, so daß ein aktiver, gerichteter Transport gegen bestehende Konzentrationsgefälle oder der Aufbau derselben ermöglicht wird. Die im ersten Raum 12 immobilisierten Zellen (Epithelzellen) sind so angeordnet, daß ihre polare Funktion bzw. aplikaler und basaler Seite bestehen bleibt, d.h. sie bilden ein intaktes, abschließendes Epithel, das diesen Gradienten erzeugt bzw. aufrechterhält.

Die hier verwendeten Membranen 15, 16, ob nun als Flachmembranen oder als Hohlfadenmembranen ausgebildet, werden auf bekannte Weise hergestellt, so daß darauf hier nicht weiter eingegangen wird.

Nachfolgend wird die Funktionsfähigkeit des Verfahrens anhand von Untersuchungen belegt.

### Zellkultur

### Nierenepithelzellen (MDCK-Zellen)

Die distale Hunde-Nierenzellinie MDCK (Madin Darby Canine Kidney) wurde für die Versuche benutzt. Diese Zellinie stellt ein etabliertes Kulturmodell für die Funktion der proximalen Nierentubili dar. Die Zellen wurden unter normalen Bedingungen in Kulturflaschen mit MEM unter Zusatz von 10 % fötalem Kälberserum, 1 % Antibiotika und 1 % Hepes kultiviert. Die Inkubation erfolgte bei 37° C und 5 % CO₂. Für die Versuche wurden die Zellen trypsiniert und mit einer Konzentration zwischen 1,5 und 2 x 10⁶ Zellen/ml in jedes Intermembran-Kompartment ausgesät. Auf Flachmembranen wurden Zellen mit einer Dichte von 80.000 Zellen/ml ausgesät (500 µl auf 0,6 mm²).

### Leberzellen (C3A-Zellen)

In diesem Fall wird die von Sussman beschriebene humane Leber-Zellinie C3A verwendet. Diese Zellinie erlaubt die Untersuchung einer Reihe von leberzelltypischen Funktionen. Die Zellen werden mit MEM unter Zusatz von 10 % fötalem Kälberserum, 1 % Antibiotika, 1 % Hepes und 1 % Pyruvat kultiviert. Nach der Trypsinierung wird eine Zelldichte zwischen 1,5 und 2 x 10⁶ Zellen/ml in jedes Intermembran-Kompartment (erster Raum 12) ausgesät. Auf Flachmembranen wurden Zellen mit einer Dichte von 100.000 Zellen/ml ausgesät (500 µl auf 0,6 mm²).

### Untersuchung des Zellwachstums auf den Trägermembranen:

Die Proliferation der Zellen auf Flachmembranen wird durch LDH- und MTT-Tests nachgewiesen. Nach 1, 2, 3, 6 und 9 Tagen Kultur auf Membranflächen von 0,6 mm² werden die verschiedenen Zellaktivitäten gemessen.

LDH: Mit dem LDH-Test wird die Menge an Laktate-Dehydrogenase bestimmt, die in der Zelle vorhanden ist. Der Test wird mit lysierten Zellen durchgeführt, so daß direkt auf die Zahl der Zellen geschlossen werden kann, die auf der Membran gewachsen sind.

Die Membranen werden zweimal mit Phosphatpuffer (PBS) gewaschen. Die auf der Membran gewachsenen Zellen werden 1,5 Stunden mit 500 µl Triton-X100 (1 % in Kulturmedium) unter Bewegung lysiert. Die Überstände werden einzeln aufgenommen und zentrifugiert (5 min, 14.000 U/min). 100 µl dieser Lösungen werden in Duplikaten in eine 96-Well-Platte gegeben und mit 100 µl LDH-Reagenz versetzt. Nach 30 Minuten Inkubation im Dunkeln bei Raumtemperatur wird die Reaktion mit 50 µl 1 N HCl gestoppt. Die optischen Dichten werden bei 492 nm gemessen.

MTT: Die mitochondrial-enzymatische Aktivität einer Dehydrogenase wird durch die Umwandlung des gelben Tetrazoliumsalzes MTT (3-[4,5-dimethylthiazol-2-yl]-2,5-diphenyltetrazoliumbromid) in nichtwasserlösliche, blaue Formazankristalle bestimmt. Auf jede Membran wird 50 µl MTT-Lösung in 500 µl Medium gegeben und 4 Stunden bei 37° C und 5 % CO₂ inkubiert. Die Überstände werden vorsichtig abgenommen und die in den Zellen gebildeten Kristalle mit 500µl Isopropanol gelöst. Nach vollständiger Auflösung der Kristalle werden die optischen Dichten bei 562 nm gemessen.

Als statistischer Test wurde die Varianz-Analyse genutzt. Eine Analyse nach Scheffe ergab signifikante Unterschiede zwischen Parametern, wenn p < 0,05 ist.

### Morphologische Untersuchungen:

Nach definierten Zeiträumen der Zellkultivierung wird der Bioreaktor geöffnet und die Faser-Verbünde mit 3 %-igem Glutaraldehyd in PBS fixiert, in kleinere Abschnitte zerteilt und weiter für verschiedene mikroskopische Untersuchungen bearbeitet.

### Lichtmikroskopie:

Nach dem Waschen der Faserabschnitte mit PBS wurden sie in einer Alkoholreihe steigender Konzentration getrocknet, mit Azeton behandelt und in Paraplast eingebettet. Die Paraffineinbettung ermöglichte die Herstellung von Schnitten am Mikrotom HM 400 (Micom, Walldorf, Deutschland). Diese Schnitte wurden dann mit Hämatoxilin und Eosin gefärbt, bevor sie mit Corbitbalsam (Hecht, Kiel) eingedeckt wurden. Diese Technik konnte nur für die PAN-Fasern genutzt werden, da PSU keine Bindung mit dem Paraffin eingeht.

### Scanning Electron Microscopy (SEM):

Nach dem Waschen der Faserabschnitte mit PBS wurden sie in einer Alkoholreihe steigender Konzentration getrocknet, bevor sie in Hexadimethyldisilasan (HMDS) zur Verdrängung von Wasserresten getaucht wurden. Nach 24 Stunden Lufttrocknung wurden die Abschnitte für 2 bis 6 Stunden in einen Exsikkator gestellt, bevor sie im EMSCOPE mit einer dünnen Kohle- und Gold-Schicht beschichtet wurden. Die Proben wurden danach mit der STEM-UNIT for EM 4.1 (Philips, Netherlands) untersucht.

### Transmission Electron Microscopy (TEM):

Die Faserabschnitte wurden in Cacodylatpuffer gewaschen, bevor sie bei 4° C mit Osmium-Säure nachfixiert wurden. Nach einem Waschvorgang mit PBS wurden die Proben in einer steigenden Alkoholreihe getrocknet, anschließend mit Propylenoxyd nachgetrocknet. Nachdem sie über Nacht in EPON:Propylenoxyd (1:1) verblieben, wurden die Proben am nächsten Tag in reines EPON eingebettet und unter Vakuum polymerisiert (600 - 800 mbar, 70° C). Ultradünne Schnitte (30 nm) wurden mit Hilfe des Ultracut E Mikrotom (Reichert-Jung, Deutschland) hergestellt. Die Schnitte wurden mit Uranylazetat gefärbt, nochmals gewaschen, getrocknet und mit Bleicitrat kontrastiert. Die Auswertung erfolgte mit dem Mikroskop EM 410 (Philips, Netherlands).

### Funktionalitätstest der Zellschichten:

### Nachweis der Albuminproduktion:

Die nach unterschiedlichen Durchflußzeiten gesammelten Medium-Proben (jeweils 1 ml) wurden mit Hilfe eines ELISA-Tests auf ihren Gehalt an Albumin untersucht. In jede der Vertiefungen einer 96-Well-Platte wurden 100 µl des ersten anti-Albumin-Antikörpers (verdünnt in Puffer A 1:5000) über Nacht immobilisiert. Nach dem Waschen mit Puffer B wurden 100 µl der Proben dazugegeben. Nach dem nächsten Waschvorgang wurde der zweite anti-Albumin-Antikörper (konjugiert mit Peroxydase, verdünnt mit Puffer B 1:8000) inkubiert. Anschließend wurde das Substrat D dazugegeben, aus dem durch die Peroxidase der Farbstoff entwickelt wird. Die optischen Dichten wurden mit dem Spektralphotometer ANTHOS 2010 bei 490 nm gemessen.

### Transportfunktion immobilisierter Nierenepithelzellen:

Mit dem Kurzschlußstrom im Experiment nach Ussing kann der aktive vom passiven elektrogenen Transport abgegrenzt werden. Das Experiment wird in einer sogenannten Ussing-Kammer durchgeführt, die zwei Elektroden enthält, durch die ein Strom von außen angelegt werden kann. Dieser Strom wird dem durch den aktiven Transport der Zellen erzeugtem Strom entgegengesetzt gerichtet und so geregelt, daß er einen gleich großen Betrag hat. Dieser "Kurzschluß" ist genau dann erzeugt, wenn die durch zwei weitere Elektroden gemessene transepitheliale Spannung Null ist. Daraus folgen zwei Konsequenzen: die hier unerwünschte elektrische Spannung ist eliminiert und der eingestellte Kurzschlußstrom stellt den Meßwert des aktiven Ionentransports dar. Die Identität der transportierten Ionensorten wird durch Agonisten oder Antagonisten mit bekannter Wirkung bestimmt, wie z.B. durch einen Kalzium-Entzug oder die Aktion von Protamin, das den transepithelialen Widerstand verringert.

### Beispiel 1:

### Wachstum von Nierenepithelzellen und Hepatozyten auf PAN- und PSU- Flachmembranen

Da ein quantitativer Nachweis des Zellwachstums auf den Membranen im Faden-in-Faden-Bioreaktor nicht möglich war, wurden Untersuchungen mit Flachmembranen durchgeführt, die vergleichbare Eigenschaften wie Hohlfadenmembranen haben und aus den gleichen Polymeren gefertigt wurden. Bei den Untersuchungen an Flachmembranen wurde zum Vergleich immer eine Kontrollmembran mitgeführt - die kommerzielle Millicell-HA-Membran (Millipore). Sie besteht aus einer Mischung von Zellulose-Azetat und -Nitrat.

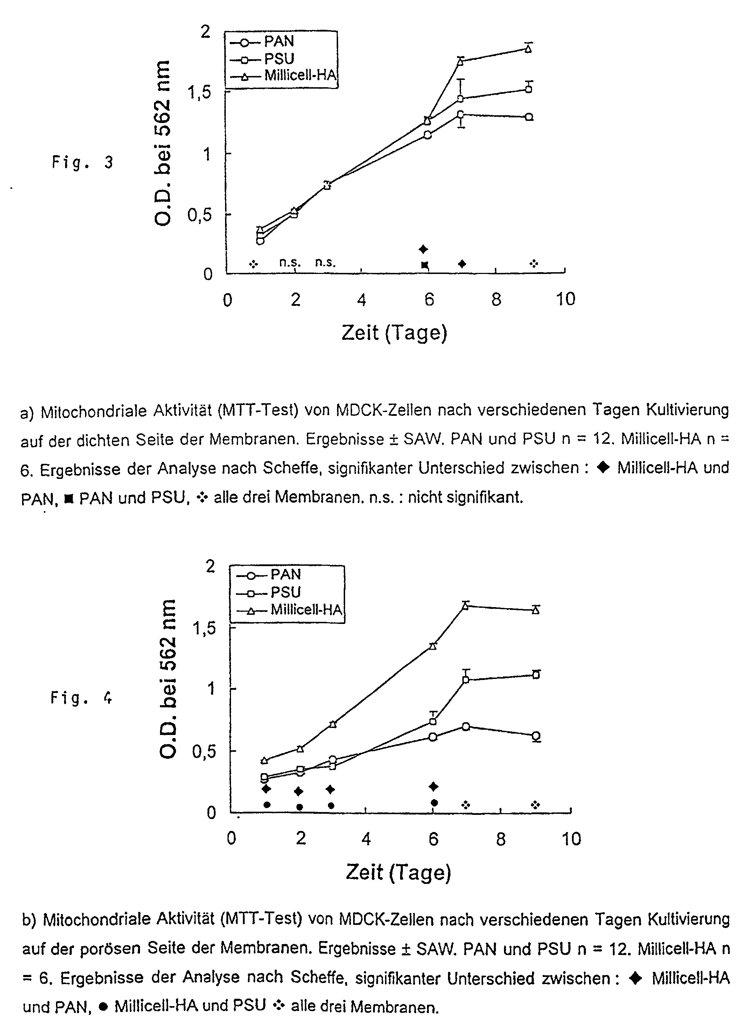

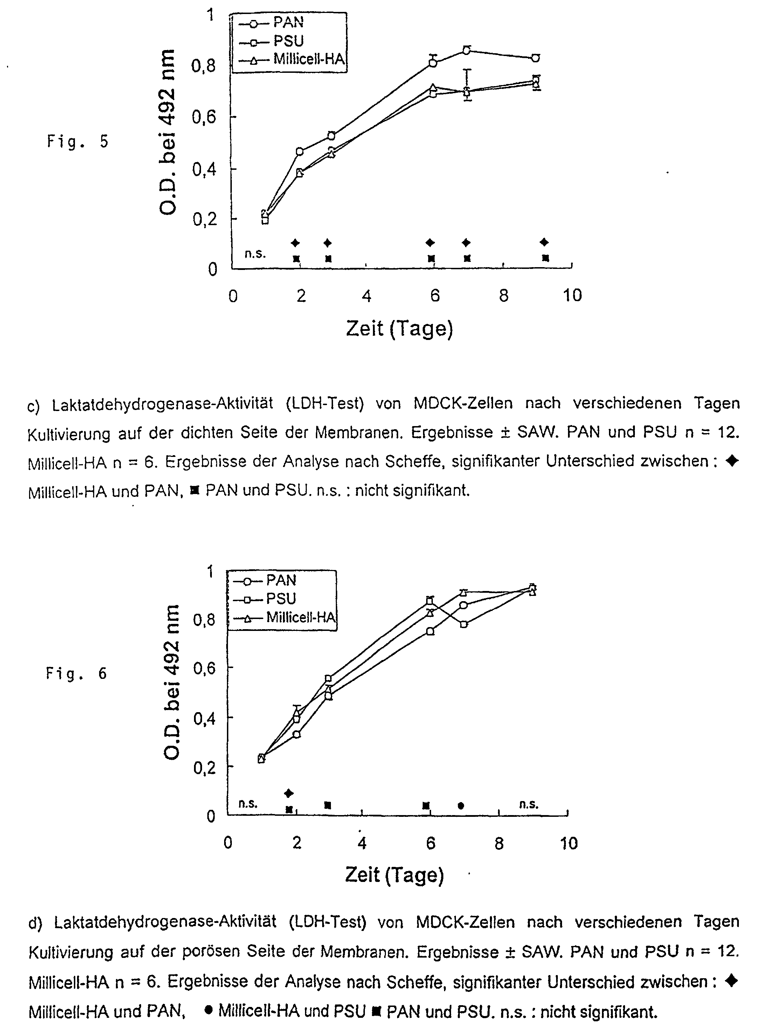

Die Proliferations-Untersuchungen von MDCK-Zellen auf den verschiedenen Oberflächen ergeben die in Fig. 3 bis 6 dargestellten Ergebnisse. Der MTT-Test zeigt, daß die Zellen sich 7 Tage lang in Kultur vermehren und die Zellzahl dann ein Maximum erreicht. Die höchsten Werte wurden für Millicell-HA gefunden, die niedrigsten für PAN. Die in den Fig. 3 bis 6 dargestellten statistischen Ergebnisse zeigen, daß sich die Membranen unabhängig von der dichten bzw. porösen Oberfläche ab 9 Tage Kultur unterscheiden.

Der LDH-Test zeigt, daß nach 6 Tagen Kultur ein Maximum der Zellzahl erreicht ist. Nach 2 Tagen unterscheidet sich die dichte Oberfläche der PAN-Membran von der der anderen beiden Membranen - bis zum Ende liefert sie die besten Ergebnisse. Die Millicell-HA- und die PSU-Membranen unterscheiden sich nicht. Auf den porösen Oberflächen erweist sich die PSU-Membran als beste Unterlage, PAN ist signifikant schlechter ab dem zweiten Tag.

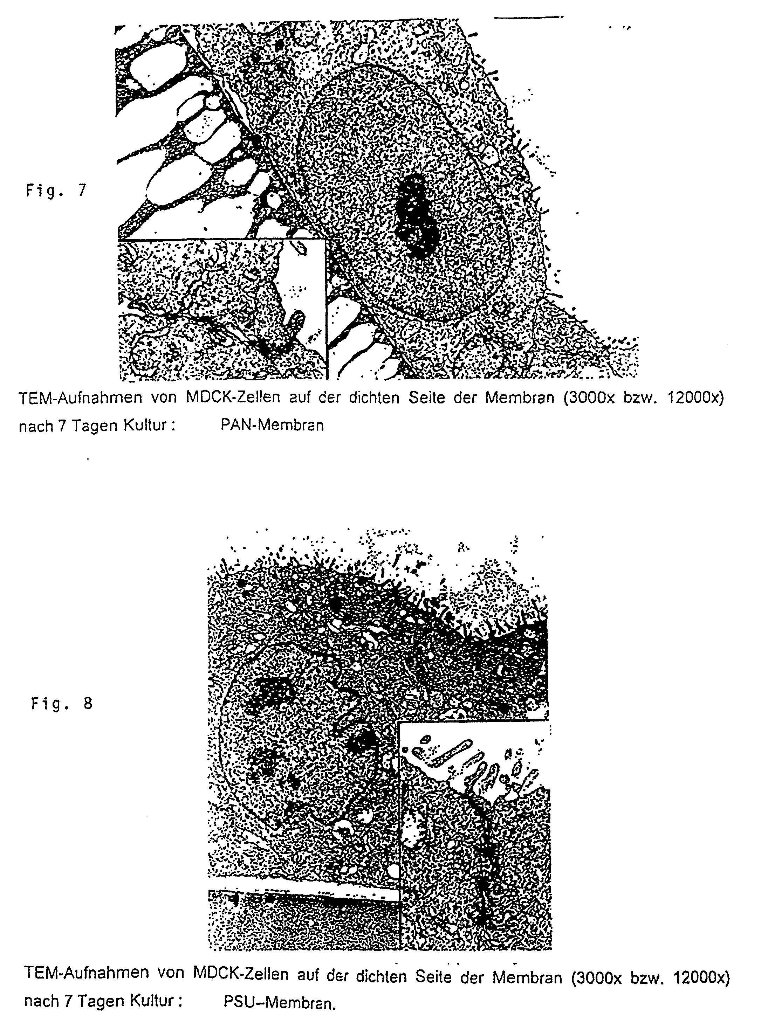

Die TEM-Untersuchungen (Fig. 7 und 8) zeigen alle typischen Merkmale dieser Zellen in Kultur auf PAN- und PSU-Membranen: Tight Junctions, Desmosomen und Mikrovillositäten sind zu sehen. Eine gute Haftung auf den PAN-Membranen mit schwachen Zell-Zell-Kontakten sowie eine geringe Haftung und ein sehr enger Zell-Zell-Kontakt auf PSU werden deutlich.

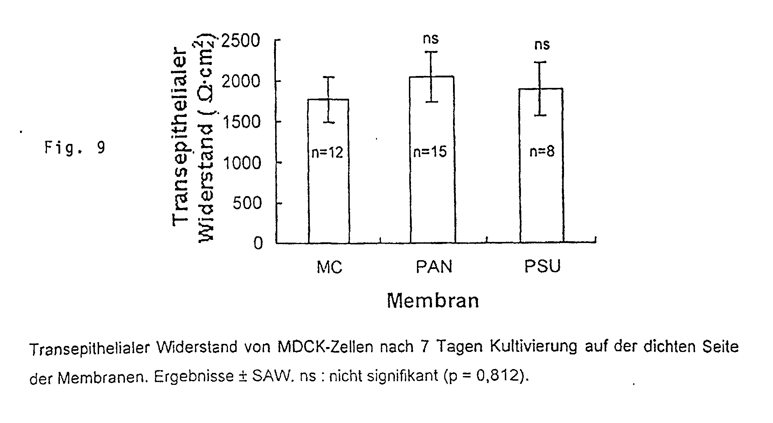

Die Funktionalität der MDCK-Zellen auf den verschiedenen dichten Oberflächen ist in der Fig. 9 dargestellt. Diese Messung wird durchgeführt, wenn die Zellen eine konfluente Zellschicht auf den Membranen gebildet haben und so die Barrierefunktionen durch die Membranen unter Kontrolle haben. Die Ergebnisse zeigen zwischen den Membranen keine Unterschiede. PAN und PSU geben vergleichbar hohe Werte wie die Kontrolle. Es wurde auch gezeigt, daß ein Kalzium-Entzug im Medium einen Widerstandsabfall verursacht. Dieser Effekt ist reversibel, das heißt, eine Zugabe von Kalzium ergibt eine neue Erhöhung des Widerstandes. Daraus kann man schließen, daß die Zellen aktiv sind und, wenn nötig, spezifische Transporte von Ionen erzwingen können.

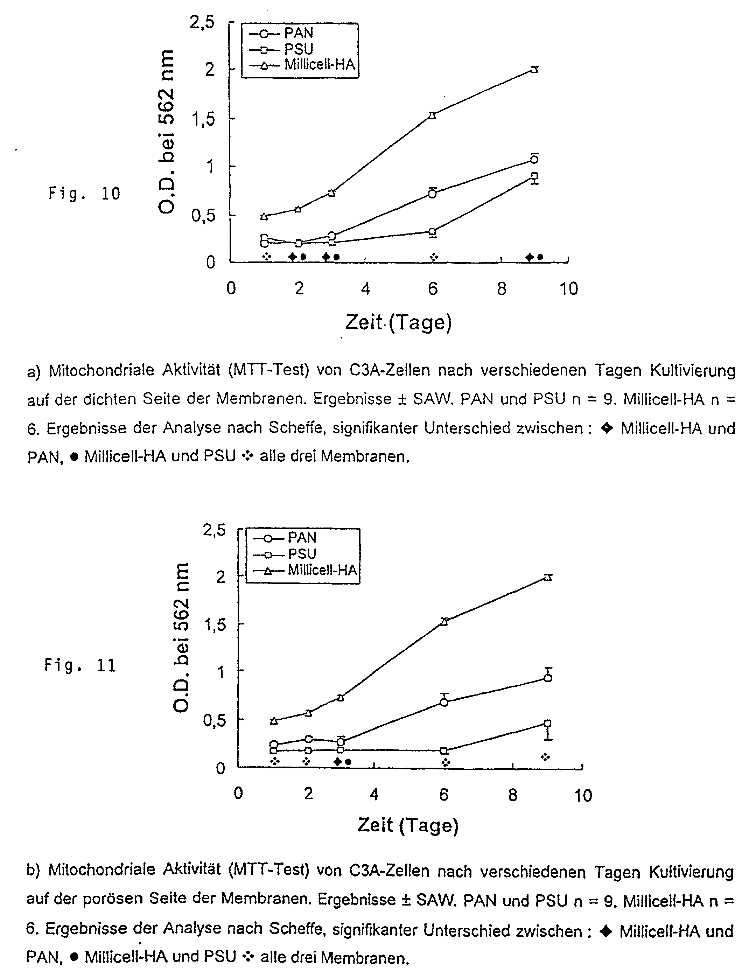

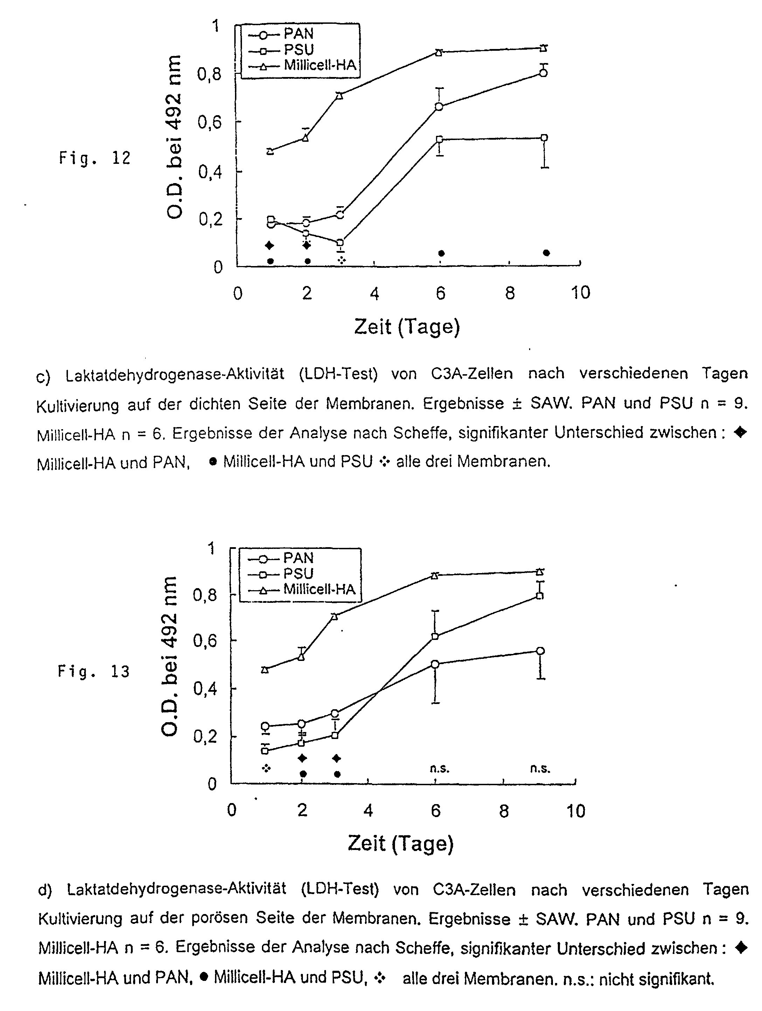

Die Proliferationsergebnisse der C3A-Zellen auf den unterschiedlichen Membranen und Oberflächen sind in den Fig. 10 bis 13 dargestellt. Aus dem MTT- und LDH-Test ist zu ersehen, daß die Millicell-HA-Membran eine bessere Vermehrung der Leberzellen ermöglicht. Es existiert kein Maximum der Proliferation nach einer gewissen Zeit. PSU scheint für diese Zellen ungeeignet - der Zeitverlust in den ersten Tagen weist auf eine schlechte Haftung der Zellen auf dieser Membran hin.

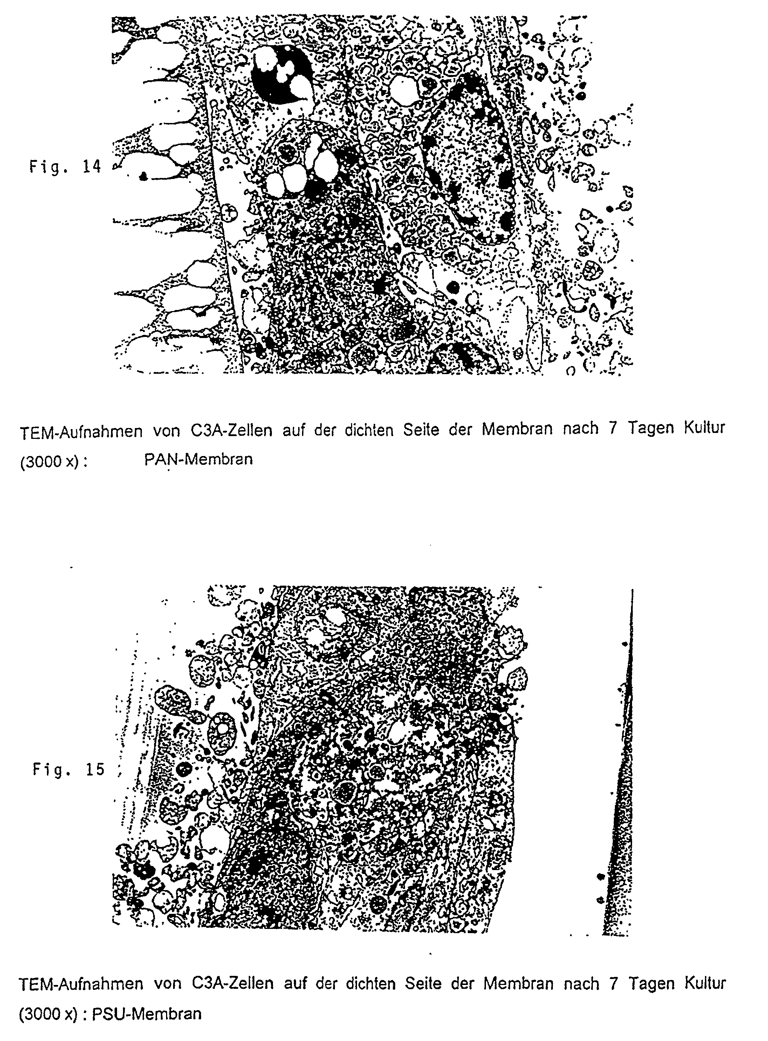

Die TEM-Untersuchungen (Fig. 14 und 15) zeigen nach 7 Tagen Kultur, daß die Zellen in Agglomeraten wachsen und Konfluenz erreicht haben. Anhand der Vielzahl an Zellresten kann geschlossen werden, daß diese Zellen eine sehr hohe Zellaktivität haben. Die schlechte Zellhaftung auf der PSU-Membrane ist wiederum zu beobachten.

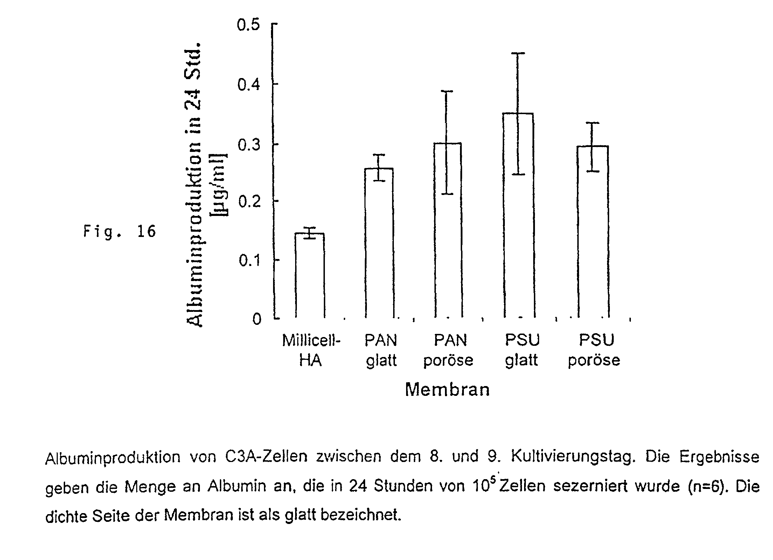

Die Funktionalität der C3A-Zellen wird anhand der Albuminproduktion zwischen dem achten und neunten Tag der Kultur bestimmt. Fig. 16 zeigt, daß die C3A-Zellen auf PAN und PSU (unabhängig von der dichten oder porösen Oberfläche) eine sehr gute Funktionalität haben, was entscheidender ist als eine höhere Proliferationsrate.

Die Ergebnisse zeigen, daß Nieren- und Leber-Zellen auf den genutzten Membranen eine gute Funktionalität besitzen, so daß die Membranen als gute Unterlage angesehen werden können. Die TEM-Untersuchungen weisen eindeutig darauf hin, daß die Zellen auf der PSU-Membran generell schlecht haften, was auf Flachmembranen problematisch sein kann, da die Zellen verloren gehen können. Für Hepatozyten spielt die Haftung möglicherweise eine untergeordnete Rolle, da diese Zellen wahrscheinlich Teilfunktionen behalten ohne zu haften.

### Beispiel 2:

### Züchtung von Nierenzellen zwischen Fäden aus Polyacrylnitril (PAN)

Die beiden asymmetrischen Polymerfäden sind aus PAN und weisen folgende Dimensionen und Charakteristika auf:
- kleiner Innenfaden: Innendurchmesser 415 µm, Wanddicke 100 µm, Wasserpermeabilität 65,8 l/m²hkPa, mittlerer Porendurchmesser D₅₀ und Cut-off D₁₀₀ konnten nicht gemessen werden
- großer Außenfaden: Innendurchmesser 750µm, Wanddicke 165 µm, Wasserpermeabilität 22,5 l/m²hkPa, mittlerer Porendurchmesser D₅₀ 0,33 µm und Cut-off D₁₀₀ 0,6 µm.

Der Bioreaktor wurde mit Ethylenoxyd sterilisiert.

### Ergebnisse:

Die lichtmikroskopische Untersuchung zeigt einen Zusammenhang zwischen der Anzahl an Zellen und der Dauer des Versuches. Die Hunde-Nieren-Zellen wachsen erst in einer Schicht mehr oder weniger kubisch und bilden nach 4 Tagen kleine dreidimensionale Gebilde, die sich mit der Zeit vermehren. Die Zellen zeigen keine Vorzugsbesiedelung für die dichte oder die poröse Oberfläche. Diese Befunde wurden durch die SEM-Untersuchungen bestätigt. Nach 14 Tagen Versuchsdauer konnten die beiden Membranen nicht mehr getrennt werden. Die typischen Merkmale dieser Zellen wie Mikrovillositäten, Tight Junctions und Desmosomen wurden durch TEM-Untersuchungen nachgewiesen. Sie sind ein Zeichen einer normalen und funktionellen Zellschicht. Eine sehr gute Zell-Unterlagen-Haftung wurde auf den PAN-Fäden festgestellt. Die verschiedenen Untersuchungen zeigen, daß die Hunde-Nierenzellen auch nach 14 Tagen Versuchsdauer eine normale und vitale Morphologie auf PAN- und PSU-Membranen vorweisen, was eine gute Funktion der Zellen belegt.

### Beispiel 3:

### Züchtung von Nierenzellen zwischen Fäden aus hydrophilisiertem Polysulfon (PSU)

Die beiden asymmetrischen Polymerfasern sind aus PSU und weisen folgende Dimensionen und Charakteristika auf:
- kleiner Innenfaden: Innendurchmesser 415 µm, Wanddicke 110 µm, Wasserpermeabilität 12,8 l/m²hkPa, mittlerer Porendurchmesser D₅₀ 0,14 µm und Cut-off D₁₀₀ 0,16 µm
- großer Außenfaden: Innendurchmesser 820 µm, Wanddicke 120 µm, Wasserpermeabilität 21,0 l/m²hkPa, mittlerer Porendurchmesser D₅₀ 0,16 µm und Cut-off D₁₀₀ 0,23 µm.

Der Bioreaktor wurde vor Gebrauch mit Wasserdampf sterilisiert.

### Ergebnisse:

Die verschiedenen Untersuchungen weisen nach, daß die MDCK-Zellen die poröse Seite der inneren Membran als Support bevorzugen. Die schlechte Zellhaftung auf den PSU-Membranen scheint jedoch die Vermehrung der Zellen nicht zu beeinträchtigen. Das sehr positive Proliferationsverhalten, unabhängig von der Haftung dieser Zellen, kann auf den begrenzten Raum und auf die Konstruktion des Bioreaktors zurückzuführen sein. Nach 7 Tagen Züchtung wird eine konfluente Zellschicht auf der porösen Seite des inneren Fadens gefunden. Die TEM-Untersuchung zeigt typische hexagonale Zellen mit Tight Junctions und Desmosomen.

Die verschiedenen Untersuchungen zeigen, daß die Hunde-Nieren-Zellen eine normale und vitale Morphologie auf PSU-Membranen vorweisen, was eine gute Funktion der Zellen belegt.

### Beispiel 4:

### Züchtung von Leberzellen zwischen Fäden aus Polyacrylnitril (PAN)

Die beiden asymmetrischen Polymerfasern bestehen aus PAN und weisen folgende Dimensionen und Charakteristika auf:
- kleiner Innenfaden: Innendurchmesser 415 µm, Wanddicke 100 µm, Wasserpermeabilität 65,8 l/m²hkPa, mittlerer Porendurchmesser D₅₀ und Cut-off D₁₀₀ wurden nicht gemessen
- großer Außenfaden: Innendurchmesser 750 µm, Wanddicke 165 µm, Wasserpermeabilität 22,5 l/m²hkPa, mittlerer Porendurchmesser D₅₀ 0,33 µm und Cut-off D₁₀₀ 0,6 µm.

Der Bioreaktor wurde mit Ethylenoxyd sterilisiert.

### Ergebnisse:

Die Leberzellen wachsen in Aggregaten und vermehren sich. Bis zu 8 Tagen Züchtung scheinen diese Zellen die poröse Seite des inneren Fadens zu bevorzugen, nach 8 Tagen wird auch die andere Membranoberfläche besiedelt. Die mikroskopischen Untersuchungen dokumentieren eine sehr gute Zell-Unterlage-Haftung. Nach 14 Tagen Züchtung präsentieren sich die Zellen groß und reich an Zytoplasma. Aggregate füllen das ganze mittlere Kompartment. Die Porosität der Unterlage scheint das Zellwachstum wenig zu beeinflussen. Die SEM-Untersuchung bestätigt, daß die Zellen nach 8 Tagen auf der porösen ebenso gut wie auch auf der dichten Seite wachsen. Mit Hilfe der TEM konnte man typische hexagonale Hepatocyten feststellen, die eine sehr gute Haftung an beiden Seiten aufweisen.

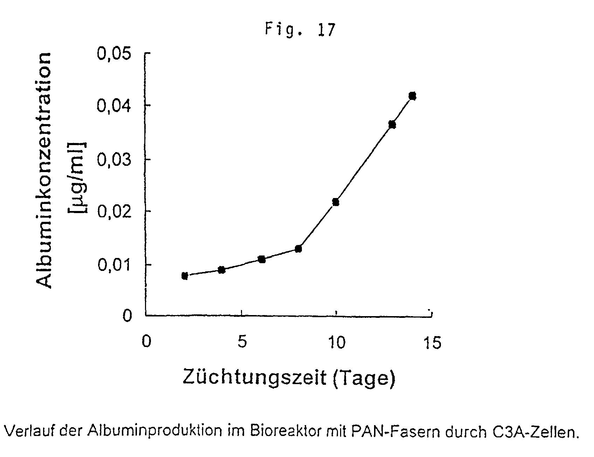

Die Funktionalität der Zellen konnte durch die Albuminbestimmung nachgewiesen werden. Die Ergebnisse sind in Fig. 17 dargestellt. Die Albuminproduktion verläuft exponentiell.

Die C3A-Leberzellen weisen eine gute Proliferation und Funktion in dem Bioreaktor auf.

### Beispiel 5:

### Züchtung von Leberzellen zwischen Fäden aus hydrophilisiertem Polysulfon (PSU)

Die beiden asymmetrischen Polymerfasern bestehen aus PSU und weisen folgende Dimensionen und Charakteristika auf:
- kleiner Innenfaden: Innendurchmesser 415 µm, Wanddicke 110 µm, Wasserpermeabilität 12,8 l/m²hkPa, mittlerer Porendurchmesser D₅₀ 0,14 µm und Cut-off D₁₀₀ 0,16 µm
- großer Außenfaden: Innendurchmesser 820 µm, Wanddicke 120 µm, Wasserpermeabilität 21,0 l/m²hkPa, mittlerer Porendurchmesser D₅₀ 0,16 µm und Cut-off D₁₀₀ 0,23 µm.

Diese Zusammenstellung erlaubt eine Dampfsterilisation des ganzen Systems.

### Ergebnisse:

Die Leberzellen wachsen in Aggregaten und vermehren sich mit der Zeit. Eine schwache Haftung ist auf PSU-Membranen zu sehen. Die Haftungsprobleme scheinen diese Zellen auch wenig zu beeinträchtigen. Sie bilden schon nach 8 Tagen Züchtung Aggregate, die praktisch das ganze mittlere Kompartment (Raum 12) füllen. Die Porosität der Unterlage scheint das Zellwachstum wenig zu beeinflussen. Die TEM-Untersuchung zeigt Tight Junctions und Desmosomen nach 8 und 12 Tagen Züchtung.

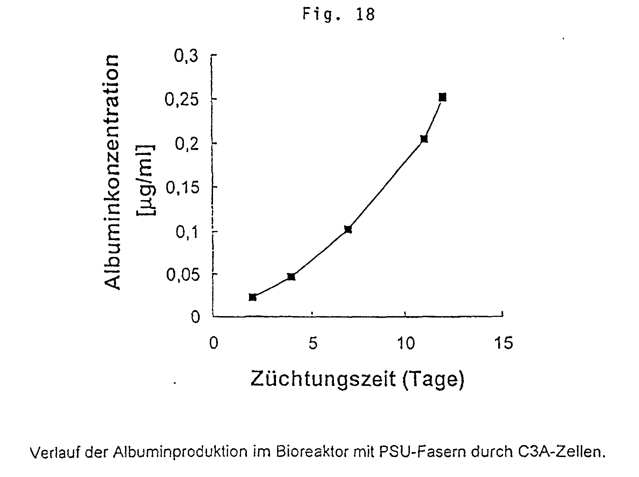

Die Funktionalität der Zellen konnte durch die Albuminbestimmung nachgewiesen werden (Fig. 18). Die Albuminproduktion ist exponentiell und besonders ausgeprägt auf PSU-Membranen, wo die Haftung schwach ist.

Die C3A-Leberzellen weisen eine gute Proliferation und Funktion in dem Bioreaktor auf. Die Konstruktion des Bioreaktors scheint der entscheidende Punkt zu sein, da die Zellen in einem engen Raum dicht beieinander wachsen können.

### Bezugszeichenliste

- 10: Vorrichtung
- 11: Gehäuse
- 12: erster Raum (Raum der Zellen)
- 120: Öffnung
- 121: Nährlösung
- 13: zweiter Raum (Medium mit Fremdstoff)
- 130: Öffnung
- 14: dritter Raum (Aufnahmeraum für abgeführte Stoffe)
- 140: Abführöffnung
- 141: abgeführte Stoffe
- 15: erste Membran
- 16: zweite Membran
- 17: Dichtung
- 18: Medium (mit Fremdstoff)
- 180: Medium (vom Fremdstoff befreit)
- 19: Pumpe
- 20: Nährlösungsvorrat
- 21: Behälter
- 22: Gasversorgung

## Patentansprüche

1. Verfahren zur Erzeugung, zum Transport und zur Eliminierung von biochemischen Stoffen mittels aktiver biologischer Zellen, **gekennzeichnet durch** folgende Schritte:
a. Einbringen von sich polar organisierenden Zellen in einen ersten Raum, der **durch** wenigstens zwei Membranen unterschiedlicher Permeabilität begrenzt ist,
b. kontinuierliches Einbringen einer Nährlösung in einen zweiten und/oder dritten Raum zum am Leben erhalten der Zellen und zur Vermehrung der Zellen,
c. Führung eines mit Fremdstoff angereicherten Mediums **durch** einen zweiten oder dritten Raum in Kontakt mit einer der beiden Membranen, die den ersten Raum vom zweiten Raum oder den ersten Raum vom dritten trennt, und eines zweiten Aufnahme-/Entgiftungsmediums **durch** den dritten oder zweiten Raum in Kontakt mit einer der beiden Membranen, die den ersten Raum vom dritten Raum oder den ersten vom zweiten Raum trennt, wobei **durch** aktive Zellfunktion der Zellen der Fremdstoff **durch** die Membran in den ersten Raum überführt und **durch** Zellstoffwechsel derart behandelt wird, daß
d. nachfolgend **durch** den Zellstoffwechsel erzeugte niedermolekulare Stoffe **durch** die andere der beiden Membranen, die den ersten Raum vom zweiten oder dritten Raum trennt, **durch** aktive Zellfunktion der Zellen überführt wird, wohingegen
e. **durch** den Zellstoffwechsel erzeugte oder entgiftete hochmolekulare Stoffe über die entgegengesetzte Membran, die den ersten Raum vom zweiten oder dritten Raum trennt, **durch** aktive Zellfunktion in den zweiten oder dritten Raum in das mit Fremdstoff belastete Medium zurücküberführt werden.

2. Verfahren nach Anspruch 1, daß die Nährlösung mit Sauerstoff und/oder Kohlendioxyd beaufschlagt wird.

3. Verfahren nach einem oder beiden der Ansprüche 1 oder 2, **dadurch gekennzeichnet, daß** die sich polar organisierenden Zellen den ersten Raum wenigstens teilweise ausfüllen.

4. Verfahren nach einem oder mehreren der Ansprüche 1 bis 3, **dadurch gekennzeichnet, daß** die sich polar organisierenden Zellen in einer wenigstens einmolekular dicken Schicht im ersten Raum ausgebildet sind.

5. Verfahren nach einem oder mehreren der Ansprüche 1 bis 4, **dadurch gekennzeichnet, daß** die erste Membran, die mit dem mit den Fremdstoffen angereicherten Medium kontaktiert wird, mikroporös ist.

6. Verfahren nach einem oder mehreren der Ansprüche 1 bis 5, **dadurch gekennzeichnet, daß** die erste Membran, die mit dem mit den Fremdstoffen angereicherten Medium kontaktiert wird, mit einer trennaktiven Schicht dem Medium zugewandt ist.

7. Verfahren nach einem oder mehreren der Ansprüche 1 bis 6, **dadurch gekennzeichnet, daß** eine Querschnittsfläche des ersten Raumes, der zwischen den beiden Membranen eingeschlossen ist, < 10 Zellendurchmesser ist.

8. Verfahren nach einem oder mehreren der Ansprüche 1 bis 6, **dadurch gekennzeichnet, daß** wenigstens eine der Membranen eine Flachmembran ist.

9. Verfahren nach einem oder mehreren der Ansprüche 1 bis 6, **dadurch gekennzeichnet, daß** wenigstens eine der Membranen eine Hohlfadenmembran ist.

10. Verfahren nach einem oder mehreren der Ansprüche 1 bis 7, 9, **dadurch gekennzeichnet, daß** beide Membranen Hohlfadenmembranen sind, wobei der erste Raum zwischen der äußeren Membranfläche einer ersten Hohlfadenmembran und der äußeren Membranfläche einer zweiten Hohlfadenmembran gebildet wird.

11. Verfahren nach einem oder mehreren der Ansprüche 1 bis 7, 9, **dadurch gekennzeichnet, daß** beide Membranen Hohlfadenmembranen sind, wobei der erste Raum zwischen der äußeren Membranfläche einer ersten Hohlfadenmembran und der inneren Membranfläche einer zweiten. Hohlfadenmembran gebildet wird.

12. Verfahren nach Anspruch 11, **dadurch gekennzeichnet, daß** die beiden Hohlfadenmembranen im wesentlichen koaxial ineinander angeordnet sind.

13. Verfahren nach einem oder mehreren der Ansprüche 1 bis 12, **dadurch gekennzeichnet, daß** wenigstens eine der Membranen eine Polymermembran ist.

14. Verfahren nach Anspruch 13, **dadurch gekennzeichnet, daß** das Polymer Polyacrilnitril (PAN) ist.

15. Verfahren nach Anspruch 13, **dadurch gekennzeichnet, daß** das Polymer Polysulfon (PSU) ist.

16. Verfahren nach Anspruch 1, **dadurch gekennzeichnet, daß** die Verfahrensschritte bei vorwählbarer Temperatur durchgeführt werden.

17. Verfahren nach Anspruch 16, **dadurch gekennzeichnet, daß** die Temperatur 37° C ist.

## Claims

1. Method for generating, transporting and eliminating biochemical substances by means of active biological cells, **characterized by** the following steps:
a. Introducing self-polar-organising cells into a first space bounded by at least two membranes with dissimilar permeability,
b. Continuously introducing a nutrient solution into a second and/or third space to keep the cells alive and to cause the cells to multiply,
c. Passing a medium enriched with an extraneous substance through a second or third space in contact with one of the two membranes separating the first space from the second space or the first space from the third, and (passing) a second absorp- tion/detoxification medium through the third or second space in contact with one of the two membranes separating the first space from the third space or the first from the second space whereby, as a result of active cell function by the cells, the extraneous substance is transferred through the membrane into the first space and is treated by cellular metabolism in such a way that
d. subsequently the low molecular weight substances formed by the cellular metabolism are transported, by the active cell function of the cells, through the other of the two membranes separating the first space from the second or third space, whereas
e. high molecular weight substances formed or detoxified by the cellular metabolism are transported back, by active cell function, through the opposite mem-brane separating the first space from the second or third space and into the second or third space into the medium contaminated with extraneous substance.

2. Process according to Claim 1, in that the nutrient solution is supplied with oxygen and/or carbon dioxide.

3. Process according to one or both of the Claims 1 or 2, **characterized in that** the self-polar-organising cells at least partially fill up the first space.

4. Process according to one or more of the Claims 1 to 3, **characterized in that** the self-polar-organising cells are configured in a layer at least one molecule thick in the first space.

5. Process according to one or more of the Claims 1 to 4, **characterized in that** the first membrane, which is in contact with the medium enriched with extraneous substances, is microporous.

6. Process according to one or more of the Claims 1 to 5, **characterized in that** the first membrane, which is in contact with the medium enriched with extraneous substances, has a layer with separatory activity facing towards the medium.

7. Process according to one or more of the Claims 1 to 6, **characterized in that** a cross-sectional surface of the first space enclosed between the two membranes is < 10 cell diameters.

8. Process according to one or more of the Claims 1 to 6, **characterized in that** at least one of the membranes is a flat membrane.

9. Process according to one or more of the Claims 1 to 6, **characterized in that** at least one of the membranes is a hollow fibre membrane.

10. Process according to one or more of the Claims 1 to 7, 9 **characterized in that** both membranes are hollow fibre membranes, whereby the first space is formed between the outer membrane surface of a first hollow fibre membrane and the outer membrane surface of a second hollow fibre membrane.

11. Process according to one or more of the Claims 1 to 7, 9 **characterized in that** both membranes are hollow fibre membranes, whereby the first space is formed between the outer membrane surface of a first hollow fibre membrane and the inner membrane surface of a second hollow fibre membrane.

12. Process according to Claim 11, **characterized in that** the two hollow fibre membranes are arranged essentially coaxially inside one another.

13. Process according to one or more of the Claims 1 to 12, **characterized in that** at least one of the membranes is a polymer membrane.

14. Process according to Claim 13, **characterized in that** the polymer is polyacrylonitrile (PAN).

15. Process according to Claim 13, **characterized in that** the polymer is polysulphone (PSU).

16. Process according to Claim 1, **characterized in that** the process steps are carried out at a pre-selectable temperature.

17. Process according to Claim 16, **characterized in that** the temperature is 37 C

## Revendications

1. Procédé pour la production, le transport et l'élimination de composés biochimiques à l'aide de cellules biologiques actives, **caractérisé par** les étapes consistant à :
a. introduire des cellules s'organisant de manière polaire dans un premier compartiment, qui est limité par au moins deux membranes de perméabilités différentes,
b. introduire en continu, une solution nutritive dans un deuxième et/ou troisième compartiment pour maintenir les cellules en vie et pour la multiplication des cellules,
c. amener un milieu enrichi de substance étrangère par un deuxième ou troisième compartiment en contact avec l'une des deux membranes, qui sépare le premier compartiment du deuxième ou le premier compartiment du troisième, et d'un deuxième milieu de fixation/détoxication par le deuxième ou troisième compartiment en contact avec l'une des deux membranes, qui sépare le premier compartiment du deuxième ou le premier compartiment du troisième, où par la fonction cellulaire active des cellules, la substance étrangère passe par la membrane dans le premier compartiment et est traitée par le métabolisme cellulaire en ce que
d. ensuite, la substance de faible poids moléculaire produite par le métabolisme cellulaire passe par l'autre des deux membranes, qui sépare le premier compartiment du deuxième ou du troisième compartiment, ce par quoi
e. les substances de poids moléculaire élevé produites ou détoxiquées par le métabolisme cellulaire repassent par la membrane opposée, qui sépare le premier compartiment du deuxième ou du troisième compartiment, par la fonction cellulaire active dans le deuxième ou troisième compartiment dans le milieu chargé de la substance étrangère.

2. Procédé selon la revendication 1, où la solution nutritive est augmenté d'oxygène et/ou de dioxyde de carbone.

3. Procédé selon l'une ou les deux revendications 1 ou 2, **caractérisé en ce que** les cellules s'organisant de manière polaire remplissent au moins partiellement le premier compartiment.

4. Procédé selon l'une ou plusieurs des revendications 1 à 3, **caractérisé en ce que** les cellules s'organisant de manière polaire constituent une couche au moins monomoléculaire dans le premier compartiment.

5. Procédé selon l'une ou plusieurs des revendications 1 à 4, **caractérisé en ce que** la première membrane, qui est en contact avec le milieu enrichi de la substance étrangère, est microporeuse.

6. Procédé selon l'une ou plusieurs des revendications 1 à 5, **caractérisé en ce que** la première membrane, qui est en contact avec le milieu enrichi de la substance étrangère, est tournée vers le milieu avec une couche active pour la séparation.

7. Procédé selon l'une ou plusieurs des revendications 1 à 6, **caractérisé en ce qu'**une section transversale du premier compartiment, qui est inclus entre les deux membranes, est < 10 diamètres cellulaires.

8. Procédé selon l'une ou plusieurs des revendications 1 à 6, **caractérisé en ce qu'**au moins une des membranes est une membrane plane.

9. Procédé selon l'une ou plusieurs des revendications 1 à 6, **caractérisé en ce qu'**au moins une des membranes est une membrane de type fibre creuse.

10. Procédé selon l'une ou plusieurs des revendications 1 à 7, 9, **caractérisé en ce que** les deux membranes sont des membranes de type fibre creuse, où le premier compartiment est formé entre la surface externe de la membrane d'une première membrane à fibre creuse et la surface externe de la membrane d'une deuxième membrane de type fibre creuse.

11. Procédé selon l'une ou plusieurs des revendications 1 à 7, 9, **caractérisé en ce que** les deux membranes sont des membranes de type fibre creuse, où le premier compartiment est formé entre la surface externe de la membrane d'une première membrane de type fibre creuse et la surface interne de la membrane d'une deuxième membrane de type fibre creuse.

12. Procédé selon la revendication 11, **caractérisé en ce que** les deux membranes de type fibre creuse sont disposées de manière essentiellement coaxiale l'une dans l'autre.

13. Procédé selon l'une ou plusieurs des revendications 1 à 12, **caractérisé en ce qu'**au moins l'une des membrane est une membrane polymère.

14. Procédé selon la revendication 13, **caractérisé en ce que** le polymère est le polyacrylonitrile (PAN).

15. Procédé selon la revendication 13, **caractérisé en ce que** le polymère est le polysulfone (PSU).

16. Procédé selon la revendication 1, **caractérisé en ce que** les étapes du procédé sont réalisées à une température pouvant être préalablement choisie.

17. Procédé selon la revendication 16, **caractérisé en ce que** la température est 37°C.
